# EUROPEAN PATENT APPLICATION

(11) **EP 2 039 342 A1**
(43) Date of publication of application: **25.03.2009**
(21) Application number: 07737280.3
(22) Date of filing: 13.06.2007
(51) Int. Cl.: A61K 8/64, A23L 1/305, A61Q 11/00

(54) **AGENT FOR MAINTAINING THE HARDNESS OF TOOTH STRUCTURE**

(30) Priority: 15.06.2006 JP 2006165591
(71) Applicant: Snow Brand Milk Products Co., Ltd., Sapporo-shi Hokkaido 065-0043 (JP)
(72) Inventor: KAWAKAMI, Hiroshi, Kawagoe-shi, Saitama 350-1142 (JP); HANADA, Nobuhiro, Tokyo 169-0072 (JP); IMAI, Susumu, Higashimurayama-shi, Tokyo 189-0022 (JP)
(74) Representative: Forstmeyer, Dietmar
(86) International application number: PCT/JP2007/000626
(87) International publication number: WO 2007/144983

(57) **Abstract**

Provided are an agent for maintaining hardness of tooth substances and a food or drink for maintaining the hardness of tooth substances, both of which include lactoferrin and iron-lactoferrin as an active ingredient. Lactoferrin and iron-lactoferrin suppress decalcification of enamel by use or ingestion thereof, and hence is effective for the prevention of dental caries.

## Description

### Technical Field

The present invention relates to an agent for maintaining hardness of tooth substances, comprising lactoferrin, which is a milk ingredient, and/or iron-lactoferrin as an active ingredient, or a food or drink for maintaining the hardness of tooth substances, including lactoferrin and/or iron-lactoferrin. The agent for maintaining the hardness of tooth substances and the food or drink for maintaining the hardness of tooth substances of the present invention can prevent the decrease in the hardness of tooth substances by the action of suppressing decalcification of enamel, and maintaining healthy teeth, mainly teeth of elderly person, and the effect of maintaining a food masticatory function for healthy dietary habit based on the healthy teeth can be obtained.

### Background Art

Dental caries is a phenomenon that hard tissue of tooth (enamel and dentin) is dissolved (decalcified). Dental caries is caused by 3 factors including microorganism, tooth, and food, specifically, the state where dental caries is highly induced by bacterial flora in the oral cavity; the state where tooth substances is highly sensitive to dental caries; and the state where dental caries is highly induced by a pattern of dietary habit. The mechanism of the occurrence of dental caries may be as follows: first, a bacterium causing dental caries (*Streptococcus mutans*) produces glucosyltransferase (GTase), which is a dental-plaque-forming enzyme; glucan, which is an insoluble, sticky polysaccharide, is synthesized using sucrose as a substrate; the synthesized glucan adheres onto the surface of teeth together with the bacterium causing dental caries, and forms a plaque; and a lactic acid bacterium (*Lactcoccus lactics* subsp. *lactis*) produces a lactic acid in the plaque, and enamel is thus decalcified. That is, occurrence of dental caries involves in many factors such as formation of a dental plaque, bacteria in the oral cavity, carbohydrates, and quality of tooth.
Thus, there have now been developed methods of preventing dental caries in consideration of those factors in various fields.

Lactoferrin is an iron-binding glycoprotein having a molecular weight of about 80 kDa and contained in mammalian milk, secretory fluid such as tears, saliva, bile, semen and the like, or secondary granules of neutrophils. X-ray analysis has revealed that the lactoferrin has a higher-order structure formed of 2 lobes.
It has also been reported that the lactoferrin has a stereostructure where a trivalent iron ion is chelately attached to the iron-binding sites (one iron-binding site is present on each lobe) in the presence of a hydrogen carbonate ion or a carbonate ion. Further, it has also been reported that the lactoferrin maintains and modulates various biological functions such as an iron-absorption-controlling action, an antimicrobial action, an anti-inflammatory action, an antioxidation action, an immunostimulatory action and the like.
Thus, attention has been drawn on the lactoferrin as a factor playing an important role in biophylaxis. As recognition of the lactoferrin as a bioactive substance is enhanced, application of the lactoferrin to food, feed, pharmaceuticals, and the like has also been under way. It is known that the lactoferrin has a pathogenic bacterium-adhesion-suppressing action, and hence is effective for prevention of dental caries (for example, see Patent Document 1). However, the action contributes just to prevent pathogenic bacteria from adhering to teeth, and it has not been disclosed whether the lactoferrin is effective for dental caries after the pathogenic bacteria have adhered to the teeth.

On the other hand, iron-lactoferrin is a substance which is able to stably hold at least 3 iron atoms per one molecule of the lactoferrin by subjecting lactoferrin to a particular treatment. For example, the followings are known: a lactoferrin powder stably holding iron which is obtained by adding an iron salt to a lactoferrin solution and increasing the pH of the solution by adding an alkali to the solution (for example, see Patent Document 2); a heat-stable lactoferrin-iron conjugate in which iron is connected to an amino group of lactoferrin via a bicarbonate ion (for example, see Patent Document 3); or an iron-lactoferrin complex obtained by adding a solution containing carbonic acid and/or bicarbonic acid and a solution containing iron to lactoferrin at a certain ratio (for example, see Patent Document 4). It has been revealed that those iron-lactoferrins have bioactivity such as anti-inflammatory effect and bone-reinforcing effect, but any study has not been tried about the effect of the iron-lactoferrins on dental caries up to the present.
[Patent Document 1] JP-A-H03-220130
[Patent Document 2] JP-A-H07-17825
[Patent Document 3] JP-A-H06-239900
[Patent Document 4] JP-A-H07-304798

### Disclosure of the Invention

### Problem to be solved by the Invention

It is an object of the present invention to provide an agent for maintaining hardness of tooth substances or a food or drink for maintaining the hardness of tooth substances, both of which include lactoferrin and/or iron-lactoferrin as an active ingredient.
The agent and the food or drink can suppress the decrease of hardness of enamel (decalcification), and thus effectively prevent dental caries, i. e. tooth decay, by directly contacting with teeth in the oral cavity.

### Means for solving the Problems

The inventors of the present invention have focused and intensively studied on lactoferrin and iron-lactoferrin, which exhibit various bioactivities, in particular high bioactivities to bones. The inventors have found that the lactoferrin and the iron-lactoferrin have effect to suppress the decrease of hardness of dental enamel caused by a pathogenic bacterium, whereby the present invention has been completed. That is, the inventors have completed an agent for maintaining the hardness of tooth substances, which includes lactoferrin and/or iron-lactoferrin both having the effect of highly preventing dental caries as an active ingredient by utilizing the ability to suppress the decrease of hardness of enamel of the lactoferrin and the iron-lactoferrin.

The lactoferrin that can be used in the present invention is made from mammalian milk such as cow milk, human milk, goat milk, and sheep milk, as a raw material. Lactoferrin contains a large amount of basic amino acids such as lysine, arginine and the like, and may be collectively called a basic protein. The basic protein can be obtained by purifying a milk material such as skimmed milk, whey or the like by using a cation-exchange resin. In the present invention, lactoferrin can be separated and purified from milk, and blended in a necessary amount. In particular, it is also possible to use a basic protein fraction containing lactoferrin obtained from a raw material such as skimmed milk or whey of cow milk.

As methods of obtaining the basic protein fraction containing lactoferrin, for example, the following methods are known: a method of obtaining the basic protein by contacting milk or a milk derived material with a cation exchangerto adsorb the basic protein, and then eluting the basic protein adsorbed to the cation exchanger with an eluate having a pH of more than 5 and an ionic strength of more than 0.5 (JP-A-H05-202098) ; a method of obtaining the basic protein fraction using an alginic acid gel (JP-A-S61-246198); a method of obtaining the basic protein fraction from whey using inorganic porous particles (JP-A-H01-86839); and a method of obtaining the basic protein fraction from milk using a sulfuric ester compound (JP-A-S63-255300). In the present invention, the basic protein fraction containing lactoferrin obtained by such methods may be used as an active ingredient.

In addition, the iron-lactoferrin that can be used in the present invention may be in the state where iron and lactoferrin are connected to each other; or the state where iron and lactoferrin are connected via another substance, in other words, any state may be preferable if the iron is not present in an ion state. For example, the above-mentioned "iron-lactoferrin conjugate" and "iron-lactoferrin complex", both of which are obtained by adding a solution containing carbonic acid and/or bicarbonic acid and a solution containing iron to lactoferrins can be given. These iron-lactoferrins may be used as an active ingredient of the present invention. Since these iron-lactoferrins have heat-resistance, there is no problem that they are even subjected to heat-treatment when adding to or preparing the agent or food or drink. In addition, these iron-lactoferrins are characterized by having no astringent taste and metallic taste of iron, and hence the problems with taste and flavor never occurs.

### Effects of the Invention

The agent for maintaining the hardness of tooth substances and the food or drink for maintaining the hardness of tooth substances of the present invention can be suppressed decalcification of dental enamel that occurs after bacteria adhere to teeth by use or ingestion thereof. Thus, the agent and the food or drink maintain the hardness of tooth substances and are effective for the prevention of dental caries.

### Best Mode for carrying out the Invention

The agent for maintaining the hardness of tooth substances of the present invention includes lactoferrin and/or iron-lactoferrin as an active ingredient. The agent can be used for pharmaceuticals and food or drink by incorporating in the form of, for example, a dentifrice, a gargle, an agent for bad breath prevention, a mouthwash, a candy, a gum, a troche and the like.
As a form of administration and a dosage thereof, it is desired that the agent be contacted with the surface of teeth in an amount of about 1 to 50 mg per adult per day dividing into several time. It should be noted that lactoferrin and iron-lactoferrin included in the present invention as an active ingredient are applied in various kinds of agents and food or drink because of their excellent bioactivity, and hence there is no problem in the safety thereof.

Hereinafter, the present invention will be described in more detail by way of Examples and Test Examples, but they are used for illustration only and are not intended to limit the present invention.

### Example 1

### (Preparation of lactoferrin and iron-lactoferrin)

A column (diameter 5 cm × height 30 cm) filled with 400 g of sulfonated Chitopearl (manufactured by Fuji Spinning Co., Ltd.) as cation-exchange resin was thoroughly washed with deionized water, and then 40 L of unsterilized skim milk (pH 6.7) was allowed to pass through the column at a flow rate of 25 ml/min. After passing, the column was thoroughly washed with deionized water, and then washed with 0.02 M carbonic acid buffer (pH 7.0) containing 0.7 M sodium chloride. After that, a fraction adsorbing to the resin was eluted with 0.02 M carbonic acid buffer (pH 7.0) containing 0.98 M sodium chloride. Subsequently, the eluted solution was desalted through a reverse osmosis (RO) membrane, and the resultant was concentrated and freeze-dried, whereby 11 g of lactoferrin powder were obtained. The lactoferrin powder contained 93 wt% of lactoferrin.
1 L of a solution containing 10 µmol of the lactoferrin powder thus obtained and 1.2 mol of sodium bicarbonate was prepared as Solution A. 1 L of a solution containing 1.5 mmol of ferric sulfate as an iron ion was prepared as Solution B. After Solution B was added to Solution A, the mixture was desalted through an ultrafiltration membrane having a molecular weight of 5,000 cuts and concentrated. The resultant was diluted with a simulated buffer solution (pH 8.9) so as to have in an iron concentration of 94 mg/100 ml, whereby obtaining iron-lactoferrin. The iron-lactoferrin thus obtained contained 0.9 g of lactoferrin and 94 mg of iron per 100 ml of the solution.

### [Test Example 1]

### (Confirmation of action of maintaining hardness of tooth substances)

The actions of maintaining the hardness of tooth substances of the lactoferrin and the iron-lactoferrin prepared in Example 1 were investigated by using an artificial oral apparatus. The apparatus includes 3 artificial oral portions, 2 pumps for sending solutions, a thermostatic bath, 3 cold stirrers, and a pH recorder. A silicon plug on which 5 stainless tubes were fixed was equipped from the upper portion, and a silicon plug on which a tube for drain and a planar pH electrode were reversely fixed was equipped from the lower portion, whereby the apparatus was hermetically sealed. The temperature inside the apparatus was kept at 37°C by circulating warm water in the water jacket of the artificial oral portion. 4 bovine enamel slabs (3.5 mm × 3.5 mm × 1.5 mm) were fixed on the Teflon holder of the surroundings of the planar pH electrode with utility wax. An HI medium containing 2.5% sucrose (1.0% on the electrode) and a bacterial suspension were dropped consecutively from the upper thereof. As a control, PBS was dropped, and as an experimental group, various milk ingredients were dropped. While the pH was continuously recorded, the dropping was continued for 15 to 18 hours, and the dropping was stopped when the pH of the control lowered to around 4 depending on formation of an artificial plaque. The artificial biofilms formed on the enamel slabs and pH electrode were treated with a 0.5 N sodium hydroxide solution, followed by centrifugation, and turbidity of the precipitate was measured at 500 nm to consider the obtained value as a bacterial cell amount. The supernatant thereof was quantified by a phenol-sulfuric acid method to consider the obtained value as a water insoluble glucan (WIG) amount. The degree of decalcification of enamel was evaluated from Vicker's hardness change (ΔH) before and after the experiment. The experiment was performed to investigate the formation of the biofilm of *S. sobrinus* ATCC33478 (OD₅₀₀=0.1), decrease of pH, and the effect of milk ingredient on decalcification of bovine enamel slabs.

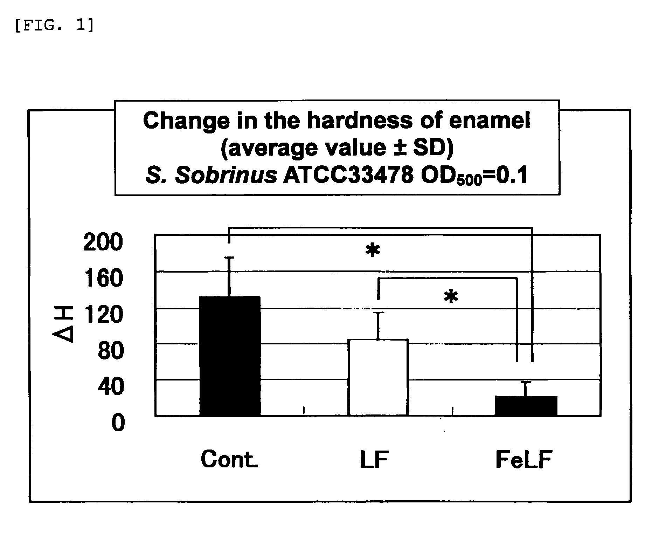

The results are shown in Fig. 1. According to Fig. 1, a significant difference (p<0.01) in the degree of decalcification of enamel between the control and both lactoferrin and iron-lactoferrin is observed. Regarding the other items, i.e., decrease of pH, the average values of the amount of bacterial cells on slabs and the amount of adhered WIG, no difference from the control was observed. That is, it was not recognized that the lactoferrin and the iron-lactoferrin exhibited an effect of suppressing the decrease of pH and an effect of suppressing formation of a biofilm in the presence of sucrose in any one of the stock strain or clinical isolate of *S. sobrinus* or *S. mutans*, but it was found that the lactoferrin and the iron-lactoferrin had the effect of maintaining the hardness of enamel.

### Example 2

### (Preparation of dentifrice for maintaining hardness of tooth substances)

The ingredients were mixed according to the formulation shown in Table 1 to prepare a cream. The cream was filled in a container to prepare a dentifrice for preventing and ameliorating periodontal disease.

**[Table 1]**

| | |
|---|---|
| Glycerin | 70.0 (wt%) |
| Silicon dioxide | 20.0 |
| Xanthan gum | 1.0 |
| Mint flavor | 1.0 |
| Titanium dioxide | 0.7 |
| Sodium fluoride | 0.3 |
| Distilled water | 6.5 |
| Lactoferrin | 0.5 |

### Example 3

### (Preparation of gargle for maintaining hardness of tooth substances)

The ingredients were mixed according to the formulation shown in Table 2 to prepare a gargle for maintaining the hardness of tooth substances.

**[Table 2]**

| | |
|---|---|
| Ethanol | 8.0 (wt%) |
| Fragrance | 0.9 |
| Sorbitol | 5.0 |
| Propylene glycol | 5.0 |
| Iron-lactoferrin | 0.1 |
| Distilled water | 81.0 |

### Example 4

### (Preparation of chewing gum for maintaining hardness of tooth substances)

The gum base was dissolved according to the formulation shown in Table 3, and stirred, and then formed into a shape to prepare a gum for maintaining the hardness of tooth substances.

**[Table 3]**

| | |
|---|---|
| Gum base | 20.0 (wt%) |
| Corn syrup | 9.0 |
| Dextrose monohydrate | 10.0 |
| Lactose | 5.0 |
| Glycerin | 5.0 |
| Saccharide | 50.0 |
| Lactoferrin | 1.0 |

### Example 5

### (Preparation of candy for maintaining hardness of tooth substances)

Sugar and starch syrup were mixed in the ratio according to the formulation shown in Table 4, stirred, and then heated up to 150°C. The mixture was cooled to 115°C, and iron-lactoferrin was added so as to contain 0.1 wt% of the iron-lactoferrin in the mixture.
The mixture was stirred, cooled on a cooling plate, and formed into a shape to prepare a candy for maintaining the hardness of tooth substances.

**[Table 4]**

| | |
|---|---|
| Sugar | 69.9 (wt%) |
| Starch syrup | 30.0 |
| Iron-lactoferrin | 0.1 |

### Brief Description of the Drawings

FIG. 1 is graph illustrating the effects of lactoferrin and iron-lactoferrin on the change in the hardness of tooth substances in Test Example 1.

## Claims

1. An agent for maintaining hardness of tooth substances, comprising lactoferrin and/or iron-lactoferrin as an active ingredient.

2. A food or drink for maintaining hardness of tooth substances, comprising lactoferrin and/or iron-lactoferrin.
